# DEMANDE DE BREVET EUROPEEN

(11) **EP 1 281 403 A1**
(43) Date de publication de la demande: **05.02.2003**
(21) Numéro de dépôt: 02291914.6
(22) Date de dépôt: 29.07.2002
(51) Int. Cl.: A61K 35/78

(54) **Preparations pharmaceutiques et/ou dietetiques contenant un extrait vegetal titre en composes actifs et des micro-organismes probiotiques**

(30) Priorité: 30.07.2001 FR 0110181
(71) Demandeur: LABORATOIRES DOLISOS, 92100 Boulogne-Billancourt (FR)
(72) Inventeur: Fabre, Pierre, 81106 Castres (FR); Fabre, Bernard, 31450 Belberaud (FR); Groubert, Alain, 07110 Vinezac (FR)
(74) Mandataire: Ahner, Francis

(57) **Abrégé**

Préparations thérapeutiques ou diététiques comprenant un extrait végétal titré en composés actifs et des micro-organismes probiotiques vivants, comme produit de combinaison pour une administration simultanée, séparée ou étalée dans le temps et à usage humain ou animal, à condition que le végétal ne soit pas le soja.

## Description

La présente invention se rapporte à des associations d'extrait végétal et de micro-organismes probiotiques, et à leurs utilisations pharmaceutiques ou diététiques.

Les plantes, qui sont répertoriées dans l'annexe 1, renferment des composés chimiques possédant des propriétés utilisables en thérapeutique ou diététique.

Ces composés chimiques sont très nombreux et peuvent appartenir à différentes classes chimiques. On peut citer notamment les composés issus du métabolisme primaire: glucides, lipides, aminoacides, peptides et protéines; les composés phénoliques: phénols, acides phénols; les terpènes et les stéroïdes, les alcaloïdes, les shikimates: coumarines, lignanes, flavonoïdes, anthocyanosides, tanins; les acétates et polyacétates: quinines, naphtodianthrones, orcinols, phloroglucinols.

Ces composés chimiques peuvent être présents sous différentes formes complexes, telles que glycosides, malonides, acétyles, aglycones ou autres.

L'intestin grêle est le lieu privilégié de l'absorption de ces composés car la surface de contact est grande, et le réseau sanguin dense.

Toutefois, la biotransformation de ces différents composés est nécessaire pour l'observation des effets bénéfiques, voire pour leur augmentation.

Cette biotransformation est effectuée par la microflore intestinale qui comprend des bactéries capables d'hydrolyser et de métaboliser lesdits composés grâce aux enzymes qu'ils sécrètent.

La diversité de la microflore intestinale joue un rôle important dans les voies de métabolisation et la biodisponibilité de ces composés.

Toutefois, il existe une grande variété métabolique individuelle pouvant dans certains cas entraîner une impossibilité de métabolisation de certains constituants, rendant de ce fait toute utilisation ou tout traitement thérapeutique inefficace.

En conséquence, il apparaît nécessaire de pallier la faible activité de certains extraits végétaux et d'améliorer leur efficacité vis-à-vis de différentes pathologies des patients peu ou pas répondeurs.

Or, les inventeurs ont trouvé de manière surprenante que certains micro-organismes probiotiques associés à des extraits végétaux titrés pouvaient résoudre ce problème.

Les probiotiques sont des "organismes vivants qui, ingérés en quantité suffisante, exercent des effets bénéfiques sur la santé allant au-delà des vertus nutritionnelles inhérentes". Les bienfaits sur la santé ne se limitent pas à l'amélioration de l'équilibre microbien au niveau de l'intestin, mais peuvent recouvrir d'autres effets. Ainsi, pour les bactéries lactiques, cinq types d'effets sont observés:
- Effets sur le tube digestif: amélioration de la digestion du lactose chez les personnes déficientes en lactase, prévention des troubles intestinaux, adhérence aux cultures de cellules intestinales, stimulation de l'immunité intestinale dans les modèles animaux, stabilisation de la maladie de Crohn et régulation de la motilité intestinale.
- Modification de la microflore: équilibre des bactéries intestinales, augmentation des bactéries fécales, diminution de l'activité des enzymes fécales, diminution de la mutagénicité fécale, soulagement des symptômes de l'intolérance au lactose et colonisation du tube digestif.
- Effet sur la diarrhée: prévention et/ou traitement des diarrhées aiguës et à rotavirus, réduction des diarrhées à rotavirus, prévention de la diarrhée associée aux antibiotiques, traitement des diarrhées récidivantes à *Clostridium difficile* et traitement des diarrhées persistantes chez l'enfant.
- Effets systémiques: stimulation de l'activité phagocytaire, stimulation de la production d'interféron γ par les cultures de cellules mononucléaires du sang humain, réduction de l'hypertension dans les modèles animaux et humains, effets bénéfiques sur le cancer superficiel de la vessie et soulagement des symptômes cliniques chez les enfants atteints de dermatite atopique.
- Effets physiologiques: production de bactériocines et effet antagoniste contre *Helicobacter pylori.*

Les Bifidobactéries, quant à elles, améliorent le transit gastro-intestinal, diminuent le risque du cancer du côlon et possèdent une activité antimicrobienne et des effets immunomodulateurs, alors que *Lactobacillus acidophilus* possède des effets hypocholestérolémiant, antimicrobien et immunomodulateur, tout en prévenant le cancer du côlon.

La présente invention a en conséquence pour objet des préparations pharmaceutiques et/ou diététiques comprenant un extrait végétal, ledit extrait étant titré de 0,1 à 30% en composés actifs et des micro-organismes probiotiques vivants, comme produit de combinaison pour une administration simultanée, séparée ou étalée dans un but thérapeutique et/ou diététique chez l'homme ou l'animal, à condition que le végétal ne soit pas le soja (Glycine max L. Merr).

Dans un mode particulier de réalisation, l'extrait végétal est choisi dans le groupe constitué par Acacia spp., Achillea millefolium L., Aconitum napellus L., Acorus calamus L., Adonis vernalis L., Aesculus hippocastanum L., Agave sisalana (Pennine), Agrimania eupatoria L., Alchemilla glabra, L. Allium cepa L., Allium sativum L., Aloes ferox Miller., Aloes vera L., Althoea officinalis L., Ammi visnaga Lam., Amorphophallus konjac K., Ananas comosus L., Anethum graveolens L., Angelica archangelica L, Apium graveolens L., Arctium lappa L., Arctostaphylos uva-ursi L., Areca catechu L., Arnica montana L., Artemisia absinthium L., Artemisia annua L., Artemisia chacunculus L., Artemisia vulgaris L., Asphalathus linearis L., Astragalus gummifer Labill, Atropa belladona L., Azadirachta indica A., Ballota nigra L., Barosma spp, Betula spp., Bixa orellana L., Borago officinalis L., Brassica juncea L., Brassica nigra L., Calamintha sylvatica Broml., Calendula officinalis L., Calophyllum inophyllum L, Camellia sinensis L., Cannabis sativa L., Capsicum spp., Carica Papaya L., Carum carvi L., Cassia angustifolia Vahl., Cassia fistula L., Catha edulis (Vahl)., Catharanthus roseus L., Centella asiatica, Centorium erythraea Rafm., Cephaelis spp., Ceratonia siliqua L., Chamaemelum nobile L., Chamomilla recutita L., Chelidonium majus L., Chrysanthellum indicum DC, Cichorium intybus L., Cimicifuga racemosa L., Cinchona spp., Cinnamomum aromaticum Nees, Cinnamomum camphora L., Cinnamomum verum J.Presl, Citrus aurantium L., Citrus spp., Claviceps purpurea L., Coffea spp., Cola spp., Colchicum autumnale L., Coleus spp., Conium maculatum L., Convallaria majalis L., Copernicia prunifera (Miller), Coriandrum sativum L., Coryllus avellana L., Crataegus monogyna Jacq., Cucurbita pepo L., Cupressus sempervirens L., Curcuma domestica Val., Curcuma xanthorrhiza Roxb., Cyamopsis tetragonolobus L., Cydonia vulgaris L., Cynara scolymus L., Cyprophila spp., Cytisus scoparius L., Datura stramonium, Derris spp., Digitalis spp., Dioscorea spp., Dipteryx odorata (Aublet), Drimia maritima L, Drosera rotundifolia L., Echinacea spp, Eleutherococcus senticosus Maxim., Ephedra spp., Equisetum arvense L., Erythroxylum spp., Eschscholtzia californica Cham., Eucalyptus globulus Labill., Eupatorium cannabinum L., Fagopyrum esculentum M., Ficus spp., Filipendula ulmaria L., Foeniculum spp., Fragaria vesca L., Fraxinus ornus L., Fucus serratus L., Fucus vesiculosus L., Fumaria officinalis L., Galium spp., Gallium odoratum L., Gelsemium sempervirens L., Gentiana lutea, Geranium robertianum L., Geum urbanum L., Ginkgo biloba L, Glechoma hederacca L., Glycyrrhiza glabra L., Gossypium spp., Guggul - Commiphora mukul, Hamamelis virginiana L., Harpagophytum procumbens (Burch)., Hedera helix L., Helleborus niger L., Hibiscus sabdariffa L., Hieracium pilosella L., Hydrastis canadensis L, Hyoscyamus niger L., Hypericum perforatum L., Hyssopus officinalis L., Ilex paraguayensis, Illicium anisatum L., Illicium verum Hook., Inula helenium L., Juglans regia, Juniperus communis L., Krameria lappacea L., Laburnum anagyroides Medikus, Laminaria spp., Lamium album L., Larrea divaricata L., Laurus nobilis L., Lavandula spp., Lawsonia inermis L., Linum usitatissimum L., Lippia citriodora H.B., Lobelia inflata L., Lophophora williamsii (Salum - Dyck), Lupinus spp, Macrocystis pyrifera Agarth, Mallotus philippinensis (Lamk.), Malva sylvestris L., Manihot esculenta Crantz, Marrubium vulgare L., Marsdenia condurango Rehb, Medicago sativa L., Melaleuca alternifolia Cheel, Melaleuca cajuputi Powell, Melaleuca quinquenervia, L. Melilotus officinalis L., Melissa officinalis L., Mentha piperata L., Menyanthes trifoliata L., Mimosa tenuiflora Willd, Myristica fragans Houtt., Myroxylon balsamum L., Nerium oleander L., Nicotiana spp., Nuphar luteum L., Ocimum basilicum L., Olea europaea L., Origanum majorana L., Origanum vulgare L., Orthosiphon aristatus (Blume) Mig, Paeonia spp., Panax ginseng C.A Meyer., Papaver somniferum L., Passiflora incarnata L., Paulina cupana, Pausinystalia yohimbe (K.Schum.), Persea americana M., Petroselium crispum (Mill.), Peumus boldus Molina., Physostigma venenosum Balf., Pilocarpus microphyllus Stafl., Pimpinella anisum L., Pinus spp, Piper methysticum Forst., Piper nigrum L., Plantago lanceolata L., Plantago major L, Plantago ovata F., Podophyllum peltatum L., Polygala senega L., Polygonum bistorta L., Potentilla erecta L, Primula veris L., Prunica granatum L., Prunus africana K, Prunus armeniaca L., Prunus laurocerasus L., Psyllium afa L., Psyllium indica L., Quercus spp., Quillaja saponaria Molina., Ranunculus ficaria L., Raphanus sativus L., Rauwolfia serpentina L, Rhammus frangula L., Rhammus purshianus DC, Rhamnus catharticus L., Rheum spp., Ribes nigrum L., Rosa canina L., Rosa gallica L., Rosmarinus officinalis L., Rubus spp., Ruscus aculeatus L., Salix alba L., Salvia spp., Sambucus nigra L., Saponaria officinalis L., Sassafras albidum Nees., Satureja montana L., Scutellaria spp., Senecio jacobea L., Senecio vulgaris L., Serenoa repens (Bartram) Small, Silybum marianum L., Simmondsia sinensis (Link), Sissymbrium officinale L., Sissymbrium officinale L., Solanum dulcamara L., Solidago virgaurea L, Sophora japonica L., Sorbus aucuparia L., Stevia rebaudiana, Strophantus spp., Strychos nux-vomica L., Styrax paralleloneurus Perkins, Styrax tonkinensis L., Syzygium aromaticum L., Tamarindus indica L., Tanacetum cinerariifolium L., Tanacetum parthenium L., Taraxacum officinale W., Taxus spp., Teucrium spp., Thaumatococcus danielli Beuth, Theobroma cacao L., Thymus serpyllum L., Thymus vulgaris L., Tilia cordata Mill., Trigonella foenum graecum L., Tropaeolum majus L., Tussilago farfara L., Urtica dioica L., Vaccinium myrtillus L., Valeriana officinalis L., Verbena officinalis L, Vinca minor L., Vitex agnus - castus L., Vitis vinifera L., Zingiber officinalis R., Ziziphus jujuba Miller

Dans un mode particulier de réalisation, les micro-organismes probiotiques sont choisis dans le groupe constitué par Aspergillus niger, Aspergillus oryzae, Bacillus coagulans, Bacillus lentus, Bacillus lincheniformis, Bacillus pumilus, Bacillus subtilis (qui ne produit pas d'antibiotique), Bacteroides amylophilus, Bacteroides capillosus, Bacteroides ruminocola, Bacteroides suis, Bifidobacterium adolescentis, Bifidobacterium animalis, Bifidobacterium bifidum, Bifidobacterium infantis, Bifidobacterium longum, Bifidobacterium thermophilum, Lactobacillus acidophilus, Lactobacillus brevis, Lactobacillus bulgaricus, Lactobacillus casei, Lactobacillus cellobiosus, Lactobacillus curvatus, Lactobacillus delbruekii, Lactobacillus fermentum, Lactobacillus lactis, Lactobacillus plantarum, Lactobacillus reuteril, Leuconostoc mesenteroides, Pediococcus acidilacticii, Pediococcus cerevisiae (damnosus), Pediococcus pentosaceus, Propionibacterium, freudenreichii, Propionibacterium shermanii, Saccharomyces cerevisiae, Streptococcus cremoris, Streptococcus diacetylactis, Streptococcus faecium, Streptococcus intermedius, Streptococcus lactis, Streptococcus thermophilus.

Au sens de la présente invention, on entend par extrait végétal titré en composés actifs, un extrait préparé comme suit: broyage du végétal puis mise en contact avec un solvant de polarité variable, comme par exemple de l'eau, de l'éthanol, un mélange eau/éthanol, de l'acétone ou de l'éther; élimination du solvant d'extraction puis purification et séchage.

Les micro-organismes probiotiques sont choisis sur la base de leurs propriétés biologiques et de leur capacité à métaboliser les constituants chimiques sélectionnés.

Ces micro-organismes peuvent être utilisés sous forme de culture disponibles dans le commerce, sous forme de suspension microbienne ou de préférence sous forme d'un lyophilisat préparé dans des conditions classiques connues de l'homme du métier.

Dans un mode de réalisation particulièrement avantageux de l'invention, ces micro-organismes sont présents à raison de 50.10⁹ à 1.10⁶ bactéries par dose unitaire et l'extrait végétal est présent à raison de 10 à 300 mg par unité de prise.

Les préparations selon l'invention comprenant l'extrait végétal et les micro-organismes peuvent être présentées sous toute forme sèche ou fluide habituellement utilisée, notamment sous forme de tablettes, de comprimés, de capsules, de gélules, de sachets ou de solutions buvables en association avec tout excipient acceptable.

L'extrait végétal et les micro-organismes probiotiques peuvent être également administrés sous forme de doses unitaires séparées, notamment des tablettes, des comprimés, des capsules, des gélules, des sachets ou des solutions buvables qui peuvent être administrées séparément ou simultanément ou de manière étalée dans le temps.

Les préparations selon l'invention permettent une métabolisation des formes combinées en forme simples actives dès la première absorption, au niveau du duodénum, zone d'absorption maximale du tube digestif.

Les exemples qui suivent illustrent l'invention sans toutefois la limiter.

### Exemple 1: Sélection des micro-organismes métabolisateurs d'un extrait d'harpagophytum

### 1) Mode opératoire:

a) Préparation de l'extrait d'harpagophytum.
   L'harpagophytum, *Harpagophytum procumbens*, est une plante qui appartient à la famille des *Pedaliaceae.* Ses racines sont utilisées traditionnellement dans le traitement des manifestations articulaires douloureuses. Ces activités sont généralement attribuées à une famille de molécules appartenant aux iridoïdes dont le produit majoritaire est l'harpagoside.
b) Incubation.
   Les micro-organismes cités en annexe 2 ont été mis en culture durant 24, 48 et 72 heures dans un milieu nutritif contenant: peptone de viande 10 g/l, extrait de viande 10 g/l, extrait de levure 5 g/l, glucose 20 g/l, acétate de sodium 5 g/l, tween80 1 g/l, phosphate dipotassique 2 g/l, citrate d'ammonium 2 g/l, sulfate de magnésium 0,2 g/l, sulfate de manganèse 0,05 g/l, et un extrait d'harpagophytum contenant de l'harpagoside.
   A chacun de ces temps, un prélèvement des cultures microbiennes est effectué, dilué de moitié dans du méthanol puis filtré. La solution méthanolique est analysée selon la technique décrite CLHP (Chromatographie Liquide Haute Performance) dans la Pharmacopée Européenne.

### 2) Résultats:

On observe pour tous les temps de prélèvements et de façon croissante une diminution du titre en harpagoside et l'apparition de trois produits non identifiés, tout particulièrement avec *Lactobacillus acidophilus*.

### Exemple 2: Sélection de micro-organismes métabolisateurs d'un extrait de Ginseng

### 1) Mode opératoire:

*Panax ginseng,* communément appelé Ginseng est une plante appartenant à la famille des Araliacées dont les racines sont utilisées comme tonique, fortifiant, dans les asthénies fonctionnelles. Les molécules auxquelles on attribue ces activités appartiennent à la famille des saponines et sont appelées des ginsennosides. Leur teneur peut varier dans la plante de 2 à 3 %.

Les racines de Ginseng sont extraites classiquement par de l'eau, de l'alcool ou par un mélange de proportions variables d'eau et d'alcool. Les ginsennosides sont les molécules dosées par différentes techniques connues de l'homme du métier dans ces différents extraits.

Les extraits de racines de Ginseng titrés en ginsennosides par chromatographie liquide haute performance (CLHP) sont mis en contact dans un milieu nutritif stérile avec chaque bactérie probiotique décrite à l'exemple 1. Les cultures sont maintenues pendant 72 heures et des prélèvements sont effectués à 24, 48 et 72 h. Ces prélèvements sont dilués avec du méthanol puis filtrés et analysés en CLHP.

### 2) Résultats:

On observe des modifications des profils chromatographiques qui prouvent la diminution des ginsennosides et l'apparition de molécules nouvelles. Ces modifications ont particulièrement lieu avec *Enterococus factium, Bifidobacterium longum, Lactobacillus casei, Lactobacillus brevis, Pediococcus pentosaceus, Propionibacterium acidi, Streptococcus thermophilus, Bifidobacterium bifidum, Lactobacillus acidophilus* et *Bifidobacterium infantis.*

### Exemple 3: Sélection de micro-organismes métabolisateurs d'un extrait de saule

### 1) Mode opératoire:

a) Préparation de l'extrait de saule.
   Les saules sont des plantes qui appartiennent à la famille des Salicacées; plusieurs espèces sont inscrites dans différentes pharmacopées, *Salix alba, Salix purpurea, Salix viminalis, Salix daphnoïdes* pour la présence de dérivés phénoliques, et de dérivés salicylés, dans leurs écorces et leurs feuilles. D'autres espèces du même genre sont également autorisées dans la mesure où la teneur en salicoside est d'au moins 1%. Cette dernière molécule est le composé le plus abondant parmi les dérivés salicylés auxquels appartiennent également la fragiline, la populine et le saliréposide. Le salicoside est un alcool salicylique glycosylé.
   Les écorces et feuilles de Saule sont utilisées dans les états fébriles, en cas de rhumatismes et de maux de tête, du fait des propriétés antalgiques et analgésiques des dérivés salicylés et en particulier du salicoside. Ce dernier est dosé par CLHP soit dans les végétaux, soit dans les extraits, obtenus par extraction avec des solvants aqueux, alcoolique ou des mélanges alcool/eau en toute proportion.
b) Incubation.
   Les extraits de saules titrés en dérivés salicylés sont mis en contact dans un milieu de culture avec chaque bactérie listée en annexe pendant 72 h. Des prélèvements effectués à 24 h, 48 h et 72 h, sont dilués dans du méthanol, filtrés et analysés en CLHP pour leur teneur en dérivés salicylés.

### 2) Résultats:

On observe une modification des chromatogrammes avec une disparition ou une nette diminution du salicoside, de la fragiline et de la populine et l'apparition de l'alcool salicylique (forme aglycone du salicoside). On peut également observer dans quelques cas une oxydation de l'alcool salicylique en la forme acide, l'acide salicylique. Ces métabolisations ont particulièrement lieu avec les probiotiques suivants: *Lactobacillus acidophilus, Lactobacillus plantarum, Streptococcus thermophilus, Streptococcus faecium, Bacteroides amylophilus* et *Bifidobacterlum animalis.*

### Exemple 4: Sélection de micro-organismes métabolisateurs d'un extrait de vigne rouge

### 1) Mode opératoire:

a) Préparation de l'extrait de vigne rouge.
   La Vigne, *Vitis vinifera* est une plante qui appartient à la famille botanique des Vitacées. Les feuilles sont utilisées pour traiter l'insuffisance veineuse périphérique et pour leurs propriétés veinotoniques sur les parois veineuses. Ces activités sont dues aux substances polyphénoliques. Ces molécules appartiennent à plusieurs sous familles des polyphénols: anthocyanes dont les molécules majoritaires sont les glucosides en 3 du cyanidol et du péonidol, acides phénoliques (dont l'acide monocafeyl-tartrique), des flavonoïdes (quercitrine, kaempferol, rutine, quercétine), des tanins hydrolysables et des proanthocyanidols ou tanins condensés.
b) Extraction.
   Les extraits végétaux réalisés sur les feuilles de vigne sont effectués avec des solvants polaires tels que l'eau, l'alcool ou un mélange eau/alcool en toute proportion. Les extraits sont analysés classiquement par un dosage colorimétrique des polyphénols totaux mais on peut également, par des techniques telles que la CLHP, doser les différents polyphénols en particulier les anthocyanes, les acides phénoliques et les flavonoïdes.
c) Incubation.
   Les extraits de vigne rouge titrés en polyphénols sont mis en contact dans un milieu nutritif avec chaque bactérie listée en page 4. Des prélèvements sont effectués aux temps 24 h, 48 h et 72 h, dilués dans du méthanol, filtrés puis analysés en CLHP.

### 2) Résultats:

On observe des modifications dans la proportion des différents pics initiaux ainsi que la disparition ou l'apparition de nouvelles molécules, preuve d'une métabolisation. Les phénomènes ont particulièrement lieu avec les micro-organismes suivants: *Enterococcus factium,* *Streptococcus thermophilus, Lactobacillus brevis, Bifidobacterium bifidum, Lactobacillus lactis, Lactobacillus plantarum* et *Lactobacillus brevis.*

## Revendications

1. Préparations pharmaceutiques et/ou diététiques comprenant un extrait végétal titré de 0,1 à 30% en composés actifs et des micro-organismes probiotiques vivants, comme produit de combinaison pour une administration simultanée, séparée ou étalée dans le temps, pour une utilisation thérapeutique ou diététique chez l'homme ou l'animal, à condition que le végétal ne soit pas le soja (Glycine max L. Merr).

2. Préparations selon la revendication 1, **caractérisées en ce que** l'extrait végétal est choisi dans le groupe Acacia spp., Achillea millefolium L., Aconitum napellus L., Acorus calamus L., Adonis vernalis L., Aesculus hippocastanum L., Agave sisalana (Pennine), Agrimania eupatoria L., Alchemilla glabra, L. Allium cepa L., Allium sativum L., Aloes ferox Miller., Aloes vera L., Althoea officinalis L., Ammi visnaga Lam., Amorphophallus konjac K., Ananas comosus L., Anethum graveolens L., Angelica archangelica L, Apium graveolens L., Arctium lappa L., Arctostaphylos uva-ursi L., Areca catechu L., Arnica montana L., Artemisia absinthium L., Artemisia annua L., Artemisia chacunculus L., Artemisia vulgaris L., Asphalathus linearis L., Astragalus gummifer Labill, Atropa belladona L., Azadirachta indica A., Ballota nigra L., Barosma spp, Betula spp., Bixa orellana L., Borago officinalis L., Brassica juncea L., Brassica nigra L., Calamintha sylvatica Broml., Calendula officinalis L., Calophyllum inophyllum L, Camellia sinensis L., Cannabis sativa L., Capsicum spp., Carica Papaya L., Carum carvi L., Cassia angustifolia Vahl., Cassia fistula L., Catha edulis (Vahl)., Catharanthus roseus L., Centella asiatica, Centorium erythraea Rafm., Cephaelis spp., Ceratonia siliqua L., Chamaemelum nobile L., Chamomilla recutita L., Chelidonium majus L., Chrysanthellum indicum DC, Cichorium intybus L., Cimicifuga racemosa L., Cinchona spp., Cinnamomum aromaticum Nees, Cinnamomum camphora L., Cinnamomum verum J.Presl, Citrus aurantium L., Citrus spp., Claviceps purpurea L., Coffea spp., Cola spp., Colchicum autumnale L., Coleus spp., Conium maculatum L., Convallaria majalis L., Copernicia prunifera (Miller), Coriandrum sativum L., Coryllus avellana L., Crataegus monogyna Jacq., Cucurbita pepo L., Cupressus sempervirens L., Curcuma domestica Val., Curcuma xanthorrhiza Roxb., Cyamopsis tetragonolobus L., Cydonia vulgaris L., Cynara scolymus L., Cyprophila spp., Cytisus scoparius L., Datura stramonium, Derris spp., Digitalis spp., Dioscorea spp., Dipteryx odorata (Aublet), Drimia maritima L, Drosera rotundifolia L., Echinacea spp, Eleutherococcus senticosus Maxim., Ephedra spp., Equisetum arvense L., Erythroxylum spp., Eschscholtzia californica Cham., Eucalyptus globulus Labill., Eupatorium cannabinum L., Fagopyrum esculentum M., Ficus spp., Filipendula ulmaria L., Foeniculum spp., Fragaria vesca L., Fraxinus ornus L., Fucus serratus L., Fucus vesiculosus L., Fumaria officinalis L., Galium spp., Gallium odoratum L., Gelsemium sempervirens L., Gentiana lutea, Geranium robertianum L., Geum urbanum L., Ginkgo biloba L, Glechoma hederacca L., Glycyrrhiza glabra L., Gossypium spp., Guggul - Commiphora mukul, Hamamelis virginiana L., Harpagophytum procumbens (Burch)., Hedera helix L., Helleborus niger L., Hibiscus sabdariffa L., Hieracium pilosella L., Hydrastis canadensis L, Hyoscyamus niger L., Hypericum perforatum L., Hyssopus officinalis L., Ilex paraguayensis, Illicium anisatum L., Illicium verum Hook., Inula helenium L., Juglans regia, Juniperus communis L., Krameria lappacea L., Laburnum anagyroides Medikus, Laminaria spp., Lamium album L., Larrea divaricata L., Laurus nobilis L., Lavandula spp., Lawsonia inermis L., Linum usitatissimum L., Lippia citriodora H.B., Lobelia inflata L., Lophophora williamsii (Salum - Dyck), Lupinus spp, Macrocystis pyrifera Agarth, Mallotus philippinensis (Lamk.), Malva sylvestris L., Manihot esculenta Crantz, Marrubium vulgare L., Marsdenia condurango Rehb, Medicago sativa L., Melaleuca alternifolia Cheel, Melaleuca cajuputi Powell, Melaleuca quinquenervia, L. Melilotus officinalis L., Melissa officinalis L., Mentha piperata L., Menyanthes trifoliata L., Mimosa tenuiflora Willd, Myristica fragans Houtt., Myroxylon balsamum L., Nerium oleander L., Nicotiana spp., Nuphar luteum L., Ocimum basilicum L., Olea europaea L., Origanum majorana L., Origanum vulgare L., Orthosiphon aristatus (Blume) Mig, Paeonia spp., Panax ginseng C.A Meyer., Papaver somniferum L., Passiflora incarnata L., Paulina cupana, Pausinystalia yohimbe (K.Schum.), Persea americana M., Petroselium crispum (Mill.), Peumus boldus Molina., Physostigma venenosum Balf., Pilocarpus microphyllus Stafl., Pimpinella anisum L., Pinus spp, Piper methysticum Forst., Piper nigrum L., Plantago lanceolata L., Plantago major L, Plantago ovata F., Podophyllum peltatum L., Polygala senega L., Polygonum bistorta L., Potentilla erecta L, Primula veris L., Prunica granatum L., Prunus africana K, Prunus armeniaca L., Prunus laurocerasus L., Psyllium afa L., Psyllium indica L., Quercus spp., Quillaja saponaria Molina., Ranunculus ficaria L., Raphanus sativus L., Rauwolfia serpentina L, Rhammus frangula L., Rhammus purshianus DC, Rhamnus catharticus L., Rheum spp., Ribes nigrum L., Rosa canina L., Rosa gallica L., Rosmarinus officinalis L., Rubus spp., Ruscus aculeatus L., Salix alba L., Salvia spp., Sambucus nigra L., Saponaria officinalis L., Sassafras albidum Nees., Satureja montana L., Scutellaria spp., Senecio jacobea L., Senecio vulgaris L., Serenoa repens (Bartram) Small, Silybum marianum L., Simmondsia sinensis (Link), Sissymbrium officinale L., Sissymbrium officinale L., Solanum dulcamara L., Solidago virgaurea L, Sophora japonica L., Sorbus aucuparia L., Stevia rebaudiana, Strophantus spp., Strychos nux-vomica L., Styrax paralleloneurus Perkins, Styrax tonkinensis L., Syzygium aromaticum L., Tamarindus indica L., Tanacetum cinerariifolium L., Tanacetum parthenium L., Taraxacum officinale W., Taxus spp., Teucrium spp., Thaumatococcus danielli Beuth, Theobroma cacao L., Thymus serpyllum L., Thymus vulgaris L., Tilia cordata Mill., Trigonella foenum graecum L., Tropaeolum majus L., Tussilago farfara L., Urtica dioica L., Vaccinium myrtillus L., Valeriana officinalis L., Verbena officinalis L, Vinca minor L., Vitex agnus - castus L., Vitis vinifera L., Zingiber officinalis R., Ziziphus jujuba Miller.

3. Préparations selon l'une quelconque des revendications 1 à 2, **caractérisées en ce que** les micro-organismes sont choisis dans le groupe constitué par Aspergillus niger, Aspergillus oryzae, Bacillus coagulans, Bacillus lentus, Bacillus lincheniformis, Bacillus pumilus, Bacillus subtilis (qui ne produit pas d'antibiotique), Bacteroides amylophilus, Bacteroides capillosus, Bacteroide ruminocola, Bacteroide suis, Bifidobacterium adolescentis, Bifidobacterium animalis, Bifidobacterium bifidum, Bifidobacterium infantis, Bifidobacterium longum, Bifidobacterium thermophilum, Lactobacillus acidophilus, Lactobacillus brevis, Lactobacillus bulgaricus, Lactobacillus casei, Lactobacillus cellobiosus, Lactobacillus curvatus, Lactobacillus delbruekii, Lactobacillus fermentum, Lactobacillus lactis, Lactobacillus plantarum, Lactobacillus reuteril, Leuconostoc mesenteroides, Pediococcus acidilacticii, Pediococcus cerevisiae (damnosus), Pediococcus pentosaceus, Propionibacterium, freudenreichii, Propionibacterium shermanii, Saccharomyces cerevisiae, Streptococcus cremoris, Streptococcus diacetylactis, Streptococcus faecium, Streptococcus intermedius, Streptococcus lactis, Streptococcus thermophilus.

4. Préparations selon l'une quelconque des revendications 1 à 3, **caractérisées en ce que** les micro-organismes probiotiques sont utilisés sous forme d'une suspension microbienne ou d'un lyophilisat.

5. Préparations selon l'une quelconque des revendications 1 à 4, **caractérisées en ce que** les micro-organismes probiotiques sont présents à une concentration comprise entre 50.10⁹ et 1.10⁶ bactéries par unité de prise.

6. Utilisation d'une préparation selon l'une quelconque des revendications 1 à 5 pour la préparation d'un produit utilisable en thérapeutique ou diététique chez l'homme ou l'animal.
